(19) Europäisches Patentamt
European Patent Office
Office européen des brevets

(11) Veröffentlichungsnummer: **0 054 698**
**B1**

(12) # EUROPÄISCHE PATENTSCHRIFT

(45) Veröffentlichungstag der Patentschrift: **20.06.84**

(51) Int. Cl.³: **C 07 C 43/315,** C 25 B 3/02 // C07C41/50

(21) Anmeldenummer: **81109003.4**

(22) Anmeldetag: **27.10.81**

(54) **4,4'-Diphenylether-dialdehyd-bis-dimethylacetal und ein Verfahren zu seiner Herstellung.**

(30) Priorität: **24.12.80 DE 3048992**

(43) Veröffentlichungstag der Anmeldung: **30.06.82 Patentblatt 82/26**

(45) Bekanntmachung des Hinweises auf die Patenterteilung: **20.06.84 Patentblatt 84/25**

(84) Benannte Vertragsstaaten: **AT BE CH DE FR GB IT LI NL SE**

(56) Entgegenhaltungen:
**EP - A - 0 011 712**
**EP - A - 0 012 240**
**DE - A - 2 912 058**

(73) Patentinhaber: **HOECHST AKTIENGESELLSCHAFT, Postfach 80 03 20, D-6230 Frankfurt am Main 80 (DE)**

(72) Erfinder: **Skaletz, Detlef, Dr., Südring 281, D-6500 Mainz (DE)**

**EP 0 054 698 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft das 4,4'-Diphenylether-dialdehyd-bis-dimethylacetal und ein Verfahren zu seiner Herstellung.

Es ist bekannt, dass die anodische Alkoxylierung von gegebenenfalls substituierten Methylbenzolen der allgemeinen Formel (I) zu den entsprechenden Benzaldehyd-dialkylacetalen der allgemeinen Formel (II) führen kann:

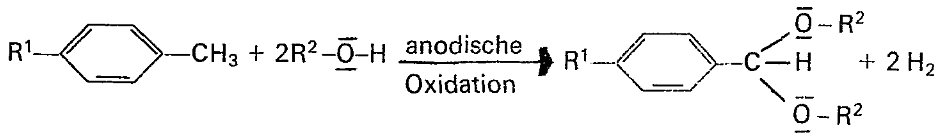


| Edukt | $R^1$ | $R^2$ | Produkt |
|---|---|---|---|
| I a | H | $CH_3$ | II a |
| b | $CH_3$ | $CH_3$ | b |
| c | $\bar{O}-CH_3$ | $CH_3$ | c |
| | | $C_2H_5$ | c' |
| d | 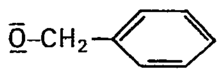 | $CH_3$ | |
| e | $\bar{O}-CH_2-CH=CH_2$ | $CH_3$ | e |
| f | 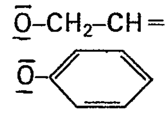 | $CH_3$ | f |
| g | $\bar{O}$-t-$C_4H_9$ | $CH_3$ | g |
| h | 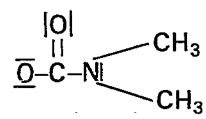 | $CH_3$ | h |

Nach dem Aufsatz «Anodic Methoxylation of Alkylbenzenes» (Anodische Methoxylierung von Alkylbenzolen) von K. Sasaki, H. Urata, K. Uneyama und S. Nagaura in Electrochimica Acta, 1967, Vol. 12, Seiten 137 bis 146 wird Toluol (Ia) an Platinelektroden in Methanol zum Benzaldehyd-dimethylacetal (IIa) (bzw. nach Hydrolyse zu dem Benzaldehyd selbst) und zum Methylbenzylether umgesetzt. Nach den Angaben in F. Beck, Elektroorganische Chemie, Verlag Chemie – Weinheim, 1974, Seite 248 soll die Ausbeute an (IIa) etwa 10% d. Th. betragen.

In der FR-A 23 51 932 wird die elektrochemische Oxidation von Methylbenzolen wie Toluol (Ia) und Xylol (Ib) beschrieben, wobei bei der Oxidation von Toluol (Ia) in einem Elektrolytsystem aus Methanol und einem Co-Lösemittel wie Methylenchlorid unter Verwendung saurer Leitelektrolyte unter nachfolgender Hydrolyse folgende Produkte erhalten werden: Methylbenzylether, Benzaldehyd und p-Methoxybenzaldehyd, daneben aber auch u.a. o-Methoxy-benzaldehyd oder p-Methoxytoluol. Die Ausbeuten an Benzaldehyd liegen bei 3,6 bis 13,2% d.Th. Bei der entsprechenden anodischen Oxidation von p-Xylol (Ib) werden – ohne Ausbeuteangaben – als Reaktionsprodukte Methyl-p-xylylether, 4-Methylbenzaldehyd, 4-Methyl-benzoesäuremethylester und 2-Methoxy-4-methyl-benzaldehyd erhalten.

Nach dem Aufsatz «Anodic Substitution and Addition Reactions» (Anodische Substitutions- und Additionsreaktionen) von S. Tsutsumi und K. Koyama in Discussions of the Faraday Society, 1968, No. 45, Seiten 247 bis 253 werden auch bei der anodischen Cyanierung von Toluol an Platinelektroden in einem System aus Methanol/NaCN kernsubstituierte Methoxylderivate des Toluols bzw. des Tolunitrils erhalten.

Es wurden in jüngerer Zeit auch bereits bemerkenswerte Selektivitätssteigerungen bei der elektrochemischen Alkoxylierung von in 4-Stellung substituierten Toluolen bekannt:

In dem Aufsatz «Nuclear Cyanation of Methylanisoles» (Kerncyanierung von Methylanisolen) von K. Yoshida, M. Shigi und T. Fueno in J. Org. Chem., 1975, Vol 40, Nr. 1, Seiten 63 bis 66 wird die Umsetzung von 4-Methoxytoluol (Ic) in einer methanolischen Lösung von NaCN oder Na-acetat beschrieben; dabei entstehen neben den jeweiligen Kernsubstitutionsprodukten auch 4-(Methoxymethyl-)anisol und in einer Material-(Strom)-Ausbeute von 15 bzw. 24% d. Th. Anisaldehyd-dimethylacetal (IIc).

A. Nilsson, U. Palmquist, T. Pettersson und A. Ronlán, «Methoxylation of Methyl-substituted Benzene and Anisole Derivatives, and the Synthesis of Aromatic Aldehydes by Anodic Oxidation» (Methoxylierung von methylsubstituierten Benzol- und Anisolderivaten und die Herstellung von aromatischen Aldehyden durch anodische Oxidation) in J. Chem. Soc., Perkin Transaktions I, 1978, Seiten 708 bis 715, gelingt es, p-Xylol (Ib) und 4-Methoxytoluol (Ic) in Methanol unter Verwendung von $NaOCH_3$ / $LiBF_4$- bzw. $NaOCH^3$-Leitelektrolyten an einer Kohleanode bei etwa 10 °C in Material(Strom)-Ausbeuten von 57% d. Th. zu (IIb) und 66% d. Th. zu (IIc) zu methoxylieren.

In der EP-A 0 011 712 (siehe auch DE-A 28 48 397) werden in 4-Stellung substituierte Benzaldehyd-dialkylacetale der allgemeinen Formel

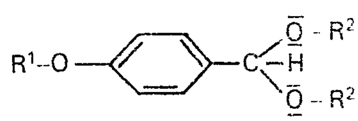

beschrieben, in der $R^1$ u.a. auch einen Phenyl- oder Benzylrest und $R^2$ einen Alkylrest mit 1 bis 4 C-Atomen bedeuten können. Diese Verbindungen werden dadurch hergestellt, dass man in 4-Stellung substituierte Methylbenzole (Toluole) der allgemeinen Formel

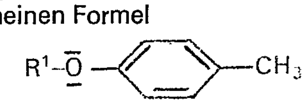

in Gegenwart eines Alkohols $R^2-\bar{O}-H$ ($R^1$ und $R^2$ haben die vorher angegebene Bedeutung) und

eines Leitsalzes elektrochemisch oxidiert. Beispiele für geeignete Leitsalze sind: Fluoride wie KF, Tetrafluorborate wie $Et_4NBF_4$, Perchlorate wie $Et_4NClO_4$, Sulfate wie $Et_4NSO_4Et$, Alkoholate wie $NaOCH_3$ und Hydroxide wie KOH. Die Mengenanteile der Komponenten sollen zwischen 5 und 50 Gew.-% für die substituierten Methylbenzole, zwischen 50 bis 95 Gew.-% für den Alkohol und zwischen 0,5 und 15 Gew.-% für das Leitsalz liegen. Als Anodenmaterialien werden Graphit, graphitgefüllte Kunststoffe und Edelmetalle, als Kathodenmaterialien Graphit, Eisen, Stahl, Blei und Edelmetalle genannt; die Stromdichten betragen 1 bis 20 $A/dm^2$, und die Elektrolysetemperatur liegt zwischen 0° und 60 °C. Im einzelnen führt die anodische Meth- bzw. Ethoxylierung von p-Xylol (Ib) in einer Material(Strom)-Ausbeute von 32 (18) % d. Th. zum 4-Methyl-benzaldehyd-dimethylacetal (IIb), von 4-Methoxytoluol (Ic) in 42,4 (22) % bis 73,1 (56,5) % zum Anisaldehyd-dimethylacetal (IIc) bzw. in 53,4 (–) % zum Anisaldehyd-diethylacetal (IIc'), von 4-Benzyloxytoluol (Id) in 62,1 (47,9) % zum 4-Benzyloxy-benzaldehyd-dimethylacetal (IId), von 4-Allyloxytoluol (Ie) in 36,3 (10,8) % zum 4-Allyloxybenzaldehyd-dimethylacetal (IIe), von 4-Phenoxytoluol (If) in 39,2 (14,3) % zum 4-Phenoxy-benzaldehyd-dimethylacetal (IIf), von 4-t-Butyloxytoluol (Ig) in 52,5 (19,2) % zum 4-t-Butyloxy-benzaldehyd-dimethylacetal (IIg) und von 4-N,N-Dimethylamino-carboxy-toluol in 40,4 (–) % zum 4-(N,N-Dimethylamino-carboxy)-benzaldehyd-dimethylacetal.

Bei dem Verfahren zur Herstellung von substituierten Benzaldehyd-dialkylacetalen gemäss der EP-A 0 012 240 werden u.a. in 4-Stellung substituierte Methylbenzole (Toluole) der allgemeinen Formel

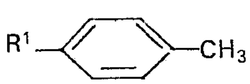

in der $R^1$ u.a. einen Aryloxy- oder Aralkoxy-Rest bedeuten kann, gelöst in einem Alkohol der Formel $R^2-\overline{O}-H$ ($R^2$ = Alkyl) und in Gegenwart eines Leitsalzes bei einer Stromdichte von 0,1 bis 50 $A/dm^2$ elektrochemisch oxidiert. Als Aryloxy- bzw. Aralkoxy-Reste werden u.a. Phenoxy-, Naphthyloxy-, Anthryloxy-, Benzyloxy- und 2-Phenyl-ethyloxy- genannt, wonach zu den in 4-Stellung substituierten Methylbenzolen dann u.a. p-Phenoxy-toluol (= 1-Methyl-4-phenoxybenzol) zählen soll. Zu den geeigneten Leitsalzen zählen Tetraethylammonium-p-toluol-sulfonat, Tetraethylammonium-ethylsulfat oder Tetramethylammonium-dimethylphosphat. Als Anodenmaterialien werden Graphit, Bleidioxid und Edelmetalle, als Kathodenmaterialien Kupfer, Nickel, Stahl, Platin und Graphit genannt. Im einzelnen führt die anodische Methoxylierung von p-Xylol (Ib) in einer Material(Strom)-Ausbeute von 64 (–) % zum 4-Methyl-benzaldehyd-dimethylacetal (IIb), von 4-Methoxytoluol (Ic) in 67 (71) % bis 95 (–) % zum 4-Methoxybenzaldehyd-dimethylacetal und von 4-t-Butyloxytoluol (Ig) in 55 (–) bis 92 (–) % zum 4-t-Butyloxy-benzaldehyd-dimethylacetal (IIg).

Keine dieser zahlreichen Veröffentlichungen enthält aber auch nur den geringsten Hinweis darauf, dass es möglich sei, aromatische Verbindungen mit 2 an einem aromatischen System ständigen Methylgruppen im Molekül gleichzeitig zu den entsprechenden aromatischen Dialdehyd-bis-dialkylacetalen zu alkoxylieren. So beschreiben beispielsweise A. Nilsson et al. (s.o.), dass die anodische Oxidation von p-Xylol (Ib) je nach Versuchsbedingung (Seite 709, rechte Spalte unten) entweder über Kernmethoxylierung zu 1-Methyl-1-methoxy-4-methyl-4-methoxy-cyclohexa-2,5-dien (III) oder über Seitenkettenmethoxylierung zum (4-Methylbenzyl)-methylether (IV), zum 4-Methyl-benzaldehyd-dimethylacetal (IIb) oder in der letzten Oxidationsstufe zum Orthoester der p-Toluylsäure (V) führt:

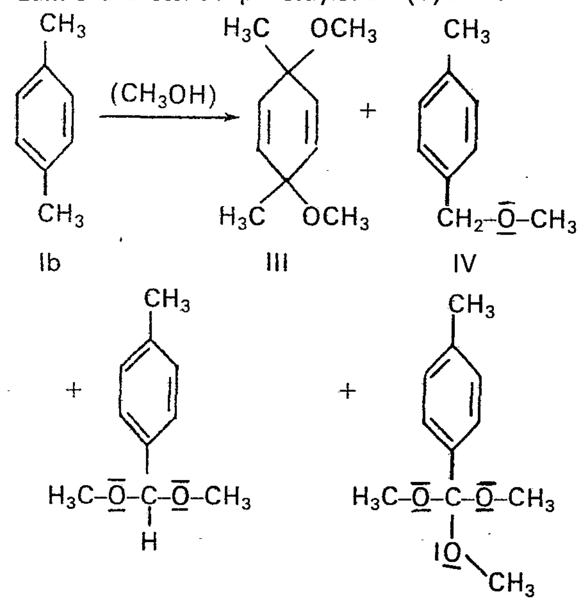


IIb V

Auch in den noch jüngeren Veröffentlichungen (DE-A 28 48 397, EP-A 0 011 712 oder EP-A 0 012 240) ist ebenfalls kein Hinweis darauf enthalten, dass bei der anodischen Methoxylierung etwa von p-Xylol (Ib) auch nur in Spuren des Terephthaldialdehyd-bis-dimethylacetal (VI)

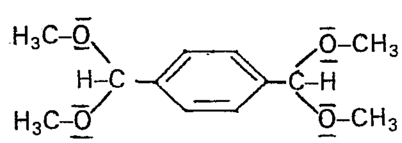

VI

entstehen könnte; selbst mit empfindlichsten analytischen Methoden – wie einer Kombination Gaschromatographie und Massenspektroskopie gemäss der FR-A 23 51 932 – konnten bei dieser Reaktion weder der Dialdehyd noch das entsprechende Bis-dimethylacetal nachgewiesen werden.

Diese Befunde decken sich mit den Ergebnissen in einem Aufsatz «Anodic Oxidation of Arylcyclopropanes» (Anodische Oxidation von Arylcyclopropanen) von T. Shono und Y. Matsumura in J. Org. Chem., 1970, Vol. 35, Nr. 12, Seiten 4157 bis 4160, wonach bei der anodischen Methoxylierung von 4-i-Propyltoluol entweder α-Methoxylierung zum 2-(p-Tolyl)-2-methoxypropan bzw. daraus das Folgeprodukt p-Isopropenyl-toluol oder α'Methoxylierung zum (p-i-Propyl-benzyl)-methylether erfolgt; demnach inhibiert eine einmal in α-Position eingetretene Methoxygruppe vollständig eine mögliche anschliessende α'-Substitution im gleichen Molekül.

Aufgabe der vorliegenden Erfindung ist es, in einer anodischen Methoxylierungsreaktion ein symmetrisches Bis-dimethylacetal, nämlich das 4,4'-Diphenylther-dialdehyd-bis-dimethylacetal, aufzufinden.

Gegenstand der Erfindung ist die neue Verbindung 4,4'-Diphenylether-dialdehyd-bis-dimethylacetal (VII)

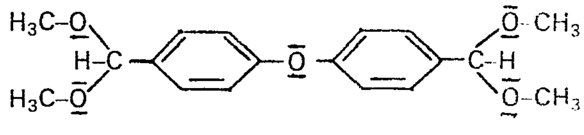

VII

Diese Verfindung wird dadurch hergestellt, dass man Di-p-tolylether (VIII) in Gegenwart von Methanol und eines Leitelektrolyten unter den nachfolgend ausgeführten Reaktionsbedingungen anodisch oxidiert:

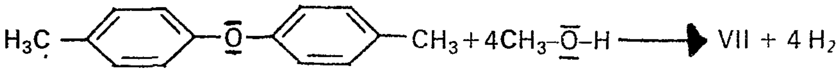

VIII

Damit gelingt es – entgegen den bisher bekannten Befunden und Erwartungen – in überraschender Weise, erstmals eine aromatische Verbindung mit 2 an einem aromatischen System ständigen Methylgruppen beidseitig, und zwar partiell und zugleich symmetrisch, anodisch so zu methoxylieren, dass als Reaktionsprodukt die Verbindung (VII) erhalten wird. Das Verfahren zur Herstellung dieser Verbindung (VII) stellt eine Modifizierung des von A. Nilsson et al. (s.o.) beschriebenen Verfahrens dar.

Das erfindungsgemässe Verfahren kann in den bekannten Druck- oder Durchflusszellen ungeteilter Bauart, möglichst unter Rühren oder Umpumpen des Elektrolyten, durchgeführt werden. Die Strömungsgeschwindigkeit des Elektrolyten ist von eher untergeordneter Bedeutung, sie kann zwischen etwa 5 cm/sec und 10 m/sec ohne wesentliche Beeinträchtigung der Reaktion variiert werden. Als Anodenmaterialien werden die in der organischen Elektrochemie üblichen Materialien wie Platin, Bleidioxid, Graphit oder glasartiger Kohlenstoff eingesetzt, bevorzugt sind metallfreie Anoden. Als Kathodenmaterialien werden die üblichen Feststoffe wie Stahl, Nickel oder Graphit verwendet, bevorzugt sind Materialien mit niedriger kathodischer Wasserstoffüberspannung. Die Form der Anoden und Kathoden ist von untergeordneter Bedeutung, es können beispielsweise Platten, Stäbe, Kugeln oder auch strukturierte Formen zum Einsatz kommen.

Die Anodenstromdichten werden im Bereich von 5 mA/cm$^2$ (0,5 A/dm$^2$) bis 500 mA/cm$^2$ (50 A/dm$^2$) variiert, bevorzugt wird ein Bereich zwischen 50 und 200 mA/cm$^2$, insbesondere ein solcher zwischen 50 und 100 mA/cm$^2$.

Als Leitelektrolyte werden im erfindungsgemässen Verfahren $NaOCH_3$, KOH, $KPF_6$, CsF, $NaBF_4$, $LiBF_4$, Tetraethylammonium-p-toluolsulfonat, $H_2SO_4$ oder $CH_3OSO_3H$ verwendet. Die Leitelektrolyte können sowohl einzeln als auch als Gemisch eingesetzt werden; bevorzugt sind im erfindungsgemässen Verfahren solche Leitelektrolyte, die nach beendeter Elektrolyse durch Zusatz eines geeigneten, möglichst weitgehend wasserfreien Hilfsstoffes in eine im Methanol unlösliche oder nicht dissoziierende Verbindung überführt werden können. Beispielsweise können Leitelektrolyte wie $H_2SO_4$ oder $NaOCH_3$ mit stöchiometrischen Mengen $NaOCH_3$ bzw. $H_2SO_4$ eine solche «inaktive» Verbindung überführt werden. Diese Möglichkeit des «Inaktivierens» des Leitelektrolyten ist insbesondere dann von Wichtigkeit, wenn die Reaktionsmischung nach abgeschlossener Elektrolyse mit dem Bis-dimethylacetal als Zwischenprodukt direkt zu chemisch abzuwandelnden Endprodukten weiterverarbeitet werden soll; in diesem Fall können dann zusätzliche Aufarbeitungsstufen entfallen. Diese Verfahrensvariante ist insbesondere von Vorteil bei der weiter unten beschriebenen Homogenhydrierung als Möglichkeit der direkten Weiterverarbeitung des Bis-dimethylacetals. Die Konzentration des Leitelektrolyten oder der Leitelektrolyte liegt im Bereich von 0,01 bis 20 Gew.-%, vorzugsweise von etwa 0,05 bis 5 Gew.-%, bezogen auf den Gesamtelektrolyten.

Das Edukt im erfindungsgemässen Verfahren, der Di-p-tolylether, kann im Elektrolyten in einer Konzentration von etwa 1 bis 40 Gew.-%, bezogen auf den Gesamtelektrolyten, anwesend sein. Als Lösemittel für das erfindungsgemässe Verfahren findet Methanol in wasserfreier oder auch in technischer Qualität mit einem geringen Wassergehalt von beispielsweise etwa 0,2 bis 0,3 Gew.-% Verwendung. Die Elektrolysetemperatur liegt zwischen 10 °C und 65 °C d.h. in etwa zwischen der Raum- und der Siedetemperatur der Elektrolytmischung.

Die Aufarbeitung des Reaktionsansatzes kann in einer der üblichen Verfahrensweisen erfolgen, beispielsweise durch Destillation oder Filtration; sie sollte jedoch vorzugsweise unter Ausschluss von Feuchtigkeit durchgeführt werden, damit das erfindungsgemäss gebildete Bis-dimethylacetal nicht zum Bis-aldehyd hydrolysiert wird. Die bei

einer solchen Aufarbeitung des Reaktionsansatzes anfallenden und damit zurückgewinnbaren Leitelektrolyte und Lösemittel können nicht nur problemlos, sondern besonders vorteilhaft in nachfolgenden Ansätzen wiederverwendet werden. In einer Variante des erfindungsgemässen Verfahrens werden – wie bereits weiter oben beschrieben – die Leitelektrolyte in «inaktive Verbindungen» überführt, so dass eine direkte Weiterverarbeitung des Reaktionsgemisches zu weiteren Produkten möglich ist.

Bei dem erfindungsgemässen Verfahren wird bei einem Stromumsatz von 8 bis 12 Faraday/mol aus dem Di-p-tolylether nahezu ausschliesslich das 4,4'-Diphenylether-dialdehyd-bis-dimethylacetal erhalten, hingegen liegt bei einem Stromumsatz von etwa 4 Faraday/mol nur ein äusserst unheitliches Produktgemisch aus 6 Verbindungen, nämlich unumgesetztem Di-p-tolylether und den Seitenkettensubstitutionsprodukten Monomethoxy-, symm. Dimeth-oxy-, unsymm. Dimethoxy-, Trimethoxy- und Tetramethoxy-di-p-tolylether vor.

Durch das erfindungsgemässe Verfahren von hohem Veredelungsgrad wird ein neuer Weg zu einer industriell wichtigen Verbindung, dem 4,4'-Bis-methoxymethyl-diphenylether (IX)

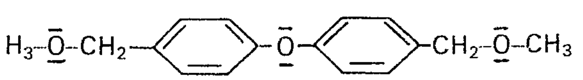

IX

eröffnet. Diese Verbindung ist insbesondere von Wichtigkeit bei der Herstellung aromatischer Polyether aus Hydroxymethyl- oder Alkyloxymethyldiarylethern (siehe z.B. DE-C 12 52 903 oder US-A 3 316 186), wobei diese aromatischen Polyether sich als Bindemittel bei der Herstellung von Gussmassen oder als Klebstoff bei der Gewinnung von Schichtkörpern eignen; eine andere bekannte Anwendung der Verbindung (IX) ist ihre in der DE-A 20 65 732 (= US-A 3 867 147) beschriebene Umsetzung mit aromatischen Diazoniumverbindungen zu lichtempfindlichen Kondensationsprodukten aus diesen beiden Komponenten. Die Herstellung von (IX) durch homogenkatalytische Hydrierung der erfindungsgemässen Verbindung 4,4'-Diphenylether-dialdehyd-bis-dimethylacetal in Methanol in Gegenwart eines mit organischen Stickstoff-Basen modifizierten, löslichen Cobaltcarbonyl-Katalysators wird in der gleichzeitig eingereichten Patentanmeldung EP-A 0 054 699 mit dem Titel «Verfahren zur katalytischen Hydrogenolyse von p-substituierten Benzaldehyddimethylacetalen zu den entsprechenden Benzylmethylether-Derivaten» ausführlich beschrieben.

In den folgenden Beispielen sind – wenn nichts anderes angegeben ist – unter %-Angaben solche zu verstehen, die auf das Gewicht bezogen sind; Gew.-Teile verhalten sich zu Vol.-Teilen wie kg zu $dm^3$.

Beispiel 1

Als Elektrolysezelle wird eine ungeteilte Durchflusszelle aus Polyethylen mit minimiertem Elektrodenabstand eingesetzt, in der die geometrischen Kathoden- und Anodenflächen 0,1 $m^2$, der Elektrodenabstand 1 mm und die Strömungsgeschwindigkeit des Elektrolyten 0,8 m/sec betragen; Einrichtungen zum Thermostatisieren, zur Probenentnahme o.ä. befinden sich ausserhalb der Zelle im Elektrolytkreislauf. Als Anode wird ein handelsüblicher mit Kunstharz imprägnierter Apparategraphit (® Diabon-N der Sigri Elektrographit GmbH, Meitingen) und als Kathode ein handelsüblicher Elektrolysegraphit (Typ-EH des gleichen Herstellers) eingesetzt. Der Elektrolyt setzt sich zusammen aus 4040 Gew.-Teilen (5100 Vol.-Teilen) technischem Methanol, 29,2 Gew.-Teilen nicht vollständig gelöstem $NaBF_4$ und 595 Gew.-Teilen Di-p-tolylether; die Molverhältnisse betragen 0,266 mol Leitsalz pro 3 mol Edukt. Die übrigen Reaktionsbedingungen sind: Temperatur von 45 °C, Stromdichte von 50 $mA/cm^2$, Zellstrom von 50 A, Zellspannung 7,3 bis 7,7 V und Stromumsatz von 9,2 F/mol entsprechend 740 Ah(= 115%).

Nach Beendigung der Reaktion wird das Methanol unter Feuchtigkeitsausschluss über eine Füllkörperkolonne (Hauptmenge unter Normaldruck, Rest im Vakuum von etwa 20 bis 40 Torr) abdestilliert, der Destillationsrückstand wird mit 2000 Vol.-Teilen trockenem Diethylether verrührt und das ausgeschiedene Leitsalz wird abgesaugt; nach Waschen des Rückstandes mit trockenem Diethylether und seiner Trocknung werden 27,6 g (= 94,5% d.Th.) des Leitsalzes zurückisoliert. Der Diethylether wird vom Reaktionsprodukt destillativ entfernt, und es werden am Dünnschichtverdampfer (Öl-Umlauftemperatur 250 °C, Ölpumpenvakuum 0,01 bis 0,2 Torr) 829,3 g destillierbare von 65,2 g nichtflüchtigen, harzartigen Bestandteilen abgetrennt.

Die gaschromatographische Analyse (GC) des Destillates [10%ig in Methanol, 1,5 m Silikon OV 225: 25% Phenyl, 25% Cyanopropyl, Methyl auf Chromosorb G AW DMS 80 bis 100 mesh (Hersteller: Fa. John Manville Products), 60 ml He/min, 80 °C Start, 8°/min] weist 90,4 Flächen-% der Verbindung (VII), dem 4,4'-Diphenylether-dialdehyd-bis-dimethylacetal aus. Neben dem Massenspektrum (Molmasse 318) belegen die Elementaranalyse

| | | | |
|---|---|---|---|
| gef.: | C 67,4% | H 6,8% | O 24,4% |
| ber.: | C 68,1% | H 6,7% | O 25,2% |

und das hochsymmetrische $^1H$-NMR-Spektrum (60 Mhz in $CDCl_3$) mit

C–H (Acetal) 12 h, S, 3,3 ppm,

C–H (Aldehyd) 2 H, S, 5,32 ppm, und

C–H (Aromat, 1.4-disubst.) 8 H, AA'BB'-System, 7,2 ppm

das Vorliegen des erfindungsgemässen Bis-dimethylacetals.

Beispiel 2

Als Elektrolysezelle wird eine ungeteilte Durchflusszelle aus Polyethylen mit minimiertem Elektrodenabstand eingesetzt; die Zelle enthält 1 Stahl VA-Endkathode, 1 Endanode aus glasartigem Kohlenstoff (® Sigradur K) und 4 bipolare Mittelelektroden aus glasartigem Kohlenstoff/Stahl VA (Hersteller der handelsüblichen Elektroden Fa. Sigri Elektrodengraphit GmbH, Meitingen). Der Elektrodenabstand wird durch 1 mm dicke Polyethylennetze definiert, die Anoden- und Kathodenflächen betragen je $5 \times 255$ cm$^2$, d.h. insgesamt je 1275 cm$^2$, die Strömungsgeschwindigkeit wird auf 0,8 m/sec eingestellt; Einrichtungen zum Thermostatisieren, zur Probenentnahme o.ä. befinden sich ausserhalb der Zelle im Elektrolykreislauf. Der Elektrolyt setzt sich zusammen aus 12750 Vol.-Teilen technischem Methanol, 30 Vol.-Teilen konz. $H_2SO_4$ und 595 Gew.-Teilen Di-p-tolylether; die übrigen Reaktionsbedingungen sind: Temperatur von 18 °C, Stromdichte von 100 mA/cm$^2$, Zellstrom von 25,5 A, Zellspannung von 34 bis 38 V und Stromumsatz von 10,6 F/mol, entsprechend 852 Ah (= 132%).

Nach Beendigung der Reaktion wird der Leitelektrolyt mit 61 Gew.-Teilen $NaOCH_3$ (in methanolischer Lösung) neutralisiert und der Elektrolyt wird gaschromatographisch nach den Angaben des Beispiels 1 aufgearbeitet und gaschromatographisch analysiert, wonach die abtrennbaren, hochsiedenden Verbindungen zu 89,7% die Verbindung (VII) enthalten.

Der neutralisierte Elektrolyt kann ohne die im Beispiel 1 angegebene Aufarbeitung nach einer Klärfiltration erfolgreich direkt beispielsweise in der in der weiter oben erwähnten Patentanmeldung EP-A 0 054 699 beschriebenen homogenen katalytischen Hydrierungsreaktion weiterverarbeitet werden.

**Patentansprüche für die Vertragsstaaten: BE, CH, DE, FR, GB, IT, LI, NL, SE**

1. 4.4'- Diphenylether -dialdehyd-bis-dimethylacetal

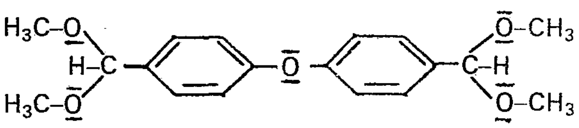

2. Verfahren zur Herstellung der Verbindung nach Anspruch 1, dadurch gekennzeichnet, dass man Di-p-tolylether

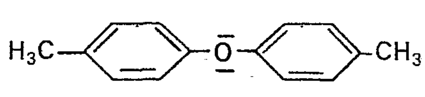

in Gegenwart von Methanol und 0,01 bis 20 Gew.-% eines Leitelektrolyten aus der Gruppe $NaOCH_3$, KOH, $KPF_6$, CsF, $NaBF_4$, $LiBF_4$, Tetraethylammonium-p-toluolsulfonat, $H_2SO_4$ und $CH_3OSO_3H$ einzeln oder als Gemisch anodisch oxidiert, wobei die Anodenstromdichte 5 bis 500 mA/cm$^2$ bei einem Stromumsatz von 8 bis 12 F/mol und die Elektrolysetemperatur 10 °C bis 65 °C betragen.

3. Verfahren nach Anspruch 2, dadurch gekennzeichnet, dass man als Anodenmaterial bei der anodischen Oxidation Platin, Bleidioxid, Graphit oder glasartigen Kohlenstoff und als Kathodenmaterial Stahl, Nickel oder Graphit einsetzt.

4. Verfahren nach Anspruch 2 oder 3, dadurch gekennzeichnet, dass als Leitelektrolyt solche Verbindungen verwendet werden, die nach beendeter Elektrolyse in eine in Methanol unlösliche oder nicht dissoziierende Verbindung überführt werden können.

5. Verfahren nach Anspruch 4, dadurch gekennzeichnet, dass als Leitelektrolyt $H_2SO_4$ oder $NaOCH_3$ verwendet wird.

6. Verfahren nach einem der Ansprüche 2 bis 5, dadurch gekennzeichnet, dass man 1 bis 50 Gew.-% Di-p-tolylether, bezogen auf den Gesamtelektrolyten, einsetzt.

**Patentansprüche für den Vertragsstaat: AT**

1. Verfahren zur Herstellung von 4,4'-Diphenylether-dialdehyd-bis-dimethylacetal, dadurch gekennzeichnet, dass man Di-p-tolylether

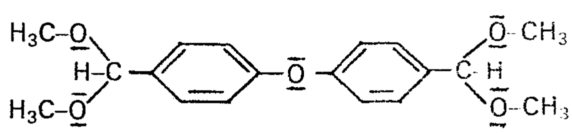

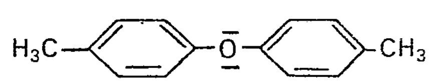

in Gegenwart von Methanol und 0,01 bis 20 Gew.-% eines Leitelektrolyten aus der Gruppe $NaOCH_3$, KOH, $KPF_6$, CsF, $NaBF_4$, $LiBF_4$, Tetraethylammonium-p-toluolsulfonat, $H_2SO_4$ und $CH_3OSO_3H$ einzeln oder als Gemisch anodisch oxidiert, wobei die Anodenstromdichte 5 bis 500 mA/cm$^2$ bei einem Stromumsatz von 8 bis 12 F/mol und die Elektrolysetemperatur 10 °C bis 65 °C betragen.

2. Verfahren nach Anspruch 1, dadurch gekennzeichnet, dass man als Anodenmaterial bei der anodischen Oxidation Platin, Bleidioxid, Graphit oder glasartigen Kohlenstoff und als Kathodenmaterial Stahl, Nickel oder Graphit einsetzt.

3. Verfahren nach Anspruch 1 oder 2, dadurch gekennzeichnet, dass als Leitelektrolyt solche Verbindungen verwendet werden, die nach beendeter Elektrolyse in eine in Methanol unlösliche oder nicht dissoziierende Verbindung überführt werden können.

4. Verfahren nach Anspruch 3, dadurch gekennzeichnet dass als Leitelektrolyt $H_2SO_4$ oder $NaOCH_3$ verwendet wird.

5. Verfahren nach einem der Ansprüche 1 bis 4, dadurch gekennzeichnet, dass man 1 bis 40 Gew.-% Di-p-tolylether, bezogen auf den Gesamtelektrolyten, einsetzt.

**Revendications pour les Etats contractants: BE, CH, DE, FR, GB, IT, LI, NL et SE**

1. 4,4'-diphényléther-dialdéhyde-bis-diméthylacétal, de formule

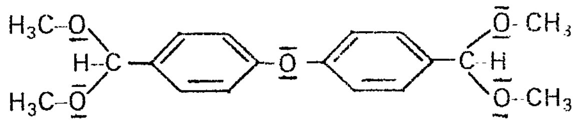

2. Procédé pour la préparation du composé selon la revendication 1, caractérisé en ce qu'on oxyde anodiquement le di-p-tolyléther de formule

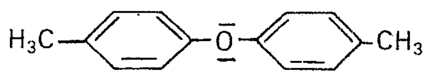

en présence de méthanol et de 0,01 à 20% en poids d'un électrolyte conducteur choisi parmi $NaOCH_3$, KOH, $KPF_6$, CsF, $NaBF_4$, $LiBF_4$, le p-toluène sulfonate de tétraéthylammonium, $H_2SO_4$ et $CH_3OSO_3H$, seuls on mélangés entre eux, la densité du courant d'anode atteignant de 5 à 500 mA/cm$^2$ pour une conversion de courant de 8 à 12 F/mole et une température d'électrolyse de 10 à 65 °C.

3. Procédé selon la revendication 2, caractérisé en ce que comme matériau d'anode pour l'oxydation anodique, on utilise le platine, le dioxyde de plomb, le graphite ou du carbone vitrifié et comme matériau de cathode on utilise l'acier, le nickel ou le graphite.

4. Procédé selon l'une des revendications 2 et 3, caractérisé en ce que comme électrolyte conducteur, on utilise des composés qui, lorsque l'électrolyse est terminée, peuvent être convertis en un composé insoluble ou non dissociable dans le méthanol.

5. Procédé selon la revendication 4, caractérisé en ce que comme électrolyte conducteur on utilise $H_2SO_4$ ou $NaOCH_3$.

6. Procédé selon l'une quelconque des revendications 2 à 5, caractérisé en ce qu'on utilise de 1 à 40% en poids de di-p-tolyléther, par rapport à l'électrolyte total.

**Revendications pour l'Etat contractant: AT**

1. Procédé pour la préparation du 4,4'-diphényl-étherdialdéhyde-bis-diméthylacétal, de formule

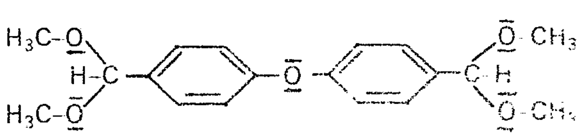

caractérisé en ce qu'on oxyde anodiquement le di-p-tolyléther, de formule

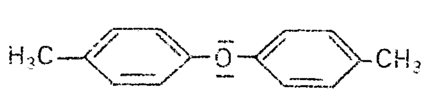

en présence de méthanol et de 0,01 à 20% en poids d'un électrolyte conducteur choisi parmi $NaOCH_3$, KOH, $KPF_6$, CsF, $NaBF_4$, $LiBF_4$, le p-toluène sulfonate de tétraéthylammonium, $H_2SO_4$ et $CH_3OSO_3H$, seuls ou mélangés entre eux, la densité du courant d'anode étant de 5 à 500 mA/cm$^2$ pour une conversion de courant de 8 à 12 F/mole et une température d'électrolyse de 10 à 65 °C.

2. Procédé selon la revendication 1, caractérisé en ce que, comme matériau d'anode pour l'oxydation anodique, on utilise le platine, le dioxyde de plomb, le graphite ou du carbone vitrifié, et comme matériau de cathode, on utilise l'acier, le nickel ou le graphite.

3. Procédé selon l'une des revendications 1 et 2, caractérisé en ce que comme électrolyte conducteur, on utilise des composés qui, lorsque l'électrolyse est terminée, peuvent être convertis en un composé insoluble ou non dissociable dans le méthanol.

4. Procédé selon la revendication 3, caractérisé en ce que comme électrolyte conducteur, on utilise $H_2SO_4$ ou $NaOCH_3$.

5. Procédé selon l'une quelconque des revendications 1 à 4, caractérisé en ce qu'on utilise de 1 à 40% en poids de di-p-tolyléther, par rapport à l'électrolyte total.

**Claims for the Contracting States: BE, CH, DE, FR, GB, IT, LI, NL and SE**

1. 4,4'- Diphenyl ether - dialdehyde - bis - dimethylacetal

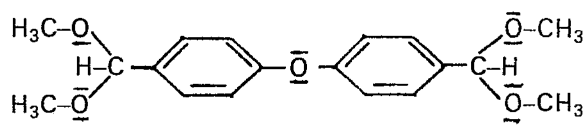

2. A process for the preparation of the compound as claimed in claim 1, which comprises anodically oxidising di-p-tolyl ether

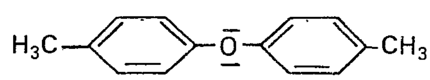

in the presence of methanol and 0,01 to 20% weight of a supporting electrolyte which is selected from the group consisting of $NaOCH_3$, KOH, $KPF_6$, CsF, $NaBF_4$, $LiBF_4$, tetraethylammonium p-toluenesulfonate, $H_2SO_4$ and $CH_3O$-$SO_3H$, individually or as a mixture, and wherein the anode current density is 5 to 500 mA/cm$^2$, the current conversion is 8 to 12 Faradays/mole, and the electrolysis temperature is 10 °C to 65 °C.

3. A process as claimed in claim 2, wherein the anode material used in the anodic oxidation is platinum, lead dioxide, graphite or vitreous carbon and the cathode material used is steel, nickel or graphite.

4. A process as claimed in claim 2 or 3, wherein the compounds used as the supporting electrolyte are of such a type that, after the electrolysis has ended, they can be converted into a compound which is insoluble or does not dissociate in methanol.

5. A process as claimed in claim 4, wherein $H_2SO_4$ or $NaOCH_3$ is used as the supporting electrolyte.

6. A process as claimed in any of claims 2 to 5, wherein 1 to 40% by weight of di-p-tolyl ether, relative to the total electrolyte, are employed.

**Claims for the Contracting State: AT**

1. A process for the preparation of 4,4'-Diphenyl ether-dialdehyde-bis-dimethylacetal

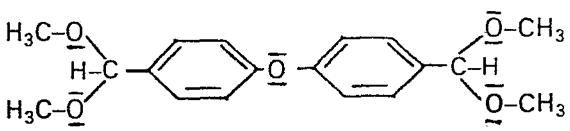

characterised in that di-p-tolyl ether

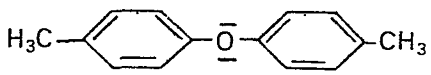

is anodically oxidised in the presence of methanol and 0,01 to 20% by weight of a supporting electrolyte which is selected from the group consisting of $NaOCH_3$, KOH, $KPF_6$, CsF, $NaBF_4$, $LiBF_4$, tetraethylammonium p-toluenesulfonate, $H_2SO_4$ and $CH_3OSO_3H$, individually or as a mixture, and wherein the anode current density is 5 to 500 mA/cm$^2$, the current conversion is 8 to 12 Faradays/mole, and the electrolysis temperature is 10 °C to 65 °C.

2. A process as claimed in claim 1, wherein the anode material used in the anodic oxidation is platinum, lead dioxide, graphite or vitreous carbon and the cathode material used is steel, nickel or graphite.

3. A process as claimed in any of claims 1 to 2, wherein the compounds used as the supporting electrolyte are of such a type that, after the electrolysis has ended, they can be converted into a compound which is insoluble or does not dissociate in methanol.

4. A process as claimed in claim 3, wherein $H_2SO_4$ or $NaOCH_3$ is used as the supporting electrolyte.

5. A process as claimed in any of claims 1 to 4, wherein 1 to 40% by weight of di-p-tolyl ether, relative to the total electrolyte, are employed.